# EUROPÄISCHE PATENTANMELDUNG

(11) **EP 1 870 079 A2**
(43) Veröffentlichungstag der Anmeldung: **26.12.2007**
(21) Anmeldenummer: 07110510.0
(22) Anmeldetag: 19.06.2007
(51) Int. Cl.: A61K 8/19, A61K 8/29, A61K 8/894, A61Q 1/02

(54) **Kosmetische Zubereitung zum Schminken der Haut**

(30) Priorität: 22.06.2006 DE 102006028549
(71) Anmelder: Beiersdorf AG, 20253 Hamburg (DE)
(72) Erfinder: Riedel, Heidi, 22529, Hamburg (DE); Bleckmann, Andreas, 22926, Ahrensburg (DE); Warnke, Katja, Hamburg, 20257 (DE); Fischer, Britta, 22880, Wedel (DE)

(57) **Zusammenfassung**

Kosmetische Zubereitung vom Typ Wasser-in-Öl, welche
a) 0,5 bis 3 Gew.-% PEG/PPG-19/19 Dimethicone,
b) 5 bis 15 Gew.-% eines oder mehrerer Pigmente sowie
c) gegebenenfalls einen oder mehrere Füllstoffe

enthält.

## Beschreibung

Die vorliegende Erfindung betrifft kosmetische Zubereitungen auf der Basis von Wasser-in-Öl-, insbesondere von Wasser-in-Siliconöl-Formulierungen, welche zum Schminken der Haut verwendet werden und mindestens einen dekorativen Bestandteil - wie Farbstoffe, Farbpigmente, Perlglanzpigmente o. ä. - enthalten. Derartige Zubereitungen werden auch als Foundations oder Gesichts-Make-up bezeichnet.

Unter Kosmetik kann man alle Maßnahmen zusammenfassen, die aus ästhetischen Gründen Veränderungen an Haut und Haaren vornehmen oder zur Körperreinigung angewendet werden. Kosmetik bedeutet also, das Körperäußere zu pflegen, zu verbessern und zu verschönern, um auf sichtbare, fühlbare und riechbare Weise sowohl den Mitmenschen als auch sich selbst zu gefallen. Schon vor Jahrtausenden wurde Kosmetik vom Menschen zu diesem Zweck angewandt. Man färbte Lippen und Gesicht, salbte sich mit wertvollen Ölen und badete in duftendem Wasser.

Im Bereich der kosmetischen Mittel sind zur Färbung des Produktes oder zur Färbung des zu behandelnden "Objektes" (Haut, Haare, Lippen) sowohl lösliche Farbmittel (im Rahmen der vorliegenden Beschreibung auch als Farbstoffe bezeichnet) und unlösliche Farbmittel (im Rahmen der vorliegenden Beschreibung auch als (Farb-) Pigmente bezeichnet) zugelassen.

Alle dekorativen Körperpflegemittel enthalten einen mehr oder weniger großen Anteil an Farbstoffen, Farbpigmenten und/oder Perlglanzpigmenten, da die farbliche Veränderung der Gesichtshaut, der Augenregion, der Lippen und/oder der Nägel der Hauptzweck dieser Produkte ist. Daneben enthalten diese Produkte üblicherweise zusätzlich weitere Inhaltsstoffe mit hautpflegender oder hautschützender Wirkung. Die Anwendung kosmetischer Make-up-Zubereitungen soll im allgemeinen die Besonderheit und Individualität einer Person unterstreichen und dabei der Betonung der persönlichen Attraktivität und der Kaschierung eventuell vorkommender Makel dienen.

Der Schminkvorgang erfolgt üblicherweise in mehreren Schritten. Zunächst wird eine flüssige Grundierung (Foundation oder Gesichts-Make-up) aufgetragen, die die Hautfarbe ausgleicht und Unregelmäßigkeiten der Haut (wie z. B. Hautunreinheiten oder Augenringe) abdeckt. Sie soll der Haut einen natürlichen und strahlenden Teint sowie ein jugendliches Aussehen verleihen.

Stärkere Unebenheiten oder Rötungen kann man mit einem hautfarbenen (Abdeck-) Stift oder Flüssig-Concealer extra kaschieren. Danach wird gewöhnlich loser oder fester Puder aufgetragen, um die Gesichthaut zu mattieren. Anschließend können die Wangen mit Rouge getönt und die Augen mit Lidschatten, Kajal, Lidstrich und/oder Mascara (Wimperntusche) geschminkt werden.

Gesichts-Make-up-Zubereitungen können vorteilhaft auch Siliconöle enthalten, weil diese Öle dazu beitragen, dass sich die Formulierungen sehr gut und gleichmäßig auf der Haut verteilen lassen und ferner der Haut einen fettfreien, weichen Glanz verleihen und dabei ein samtigweiches Hautgefühl hinterlassen.

Natürlich ist dem Fachmann eine Reihe von Möglichkeiten bekannt, Wasser-in-Siliconöl-Emulsionen (W/S-Emulsionen) herzustellen, d. h. Emulsionen, bei denen als Ölkomponenten hauptsächlich oder ausschließlich Siliconöle eingesetzt werden.

Ein Nachteil von W/S-Emulsionen des Standes der Technik ist aber, dass diese häufig nicht langzeitstabil sind. Ebenso wie Wasser-in-Öl-Emulsionen neigen auch W/S-Emulsionen z. B. zur Instabilität, wenn sie mit Glas in Berührung kommen. Üblicherweise scheidet sich bereits nach wenigen Wochen Öl aus der Zubereitung ab. Des Weiteren zeigen auch W/S-Emulsionen, die auf Dimethicone Copolyol oder Cetyl Dimethicone Copolyol basieren, bei einer Lagerdauer von mehreren Monaten und/oder bei höheren Temperaturen (wie sie z. B. bei einer Aufbewahrung im Auto leicht erreicht werden) starke Ölabscheidungen. Diese Effekte werden durch den Zusatz von Pigmenten noch verstärkt.

Aufgabe der vorliegenden Erfindung war also, kosmetische Wasser-in-Öl-, insbesondere Wasser-in-Siliconöl-Emulsionen zu finden, die die Nachteile des Standes der Technik nicht aufweisen und die eine genügend hohe Stabilität aufweisen, um in üblicher Weise - insbesondere auch in Glasbehältern - verpackt, gelagert und in den Handel gebracht werden zu können.

Überraschend hat sich gezeigt, dass eine
kosmetische Zubereitung vom Typ Wasser-in-Öl, welche
a) 0,5 bis 3 Gew.-% PEG/PPG-19/19 Dimethicone,
b) 5 bis 15 Gew.-% eines oder mehrerer Pigmente sowie
c) gegebenenfalls einen oder mehrere Füllstoffe enthält,
diese Aufgaben zu lösen vermag.

Die erfindungsgemäßen Zubereitungen stellen in jeglicher Hinsicht überaus befriedigende Präparate dar, welche auch bei höherer Temperatur und längerer Lagerdauer stabil bleiben und keine Ölabscheidung zeigen. Die erfindungsgemäßen Zubereitungen eignen sich insbesondere zum Abdecken von Hautunreinheiten und/oder Augenringen, zur Kaschierung kleiner Fältchen, zur Erreichung eines ebenmäßigen, dabei natürlichen und strahlenden Teints und eines jugendlichen Aussehens. Dabei ist der jeweils erzielte Effekt überraschend langanhaltend.

Zur Anwendung werden die erfindungsgemäßen Zubereitungen in der für Kosmetika üblichen Weise auf die Haut in ausreichender Menge aufgebracht. Sie können wie üblich zusammengesetzt sein und zur Pflege der Haut und als Schminkprodukt in der dekorativen Kosmetik dienen.

Die kosmetischen Zubereitungen gemäß der Erfindung stellen bevorzugt Emulsionen dar.

PEG/PPG-19/19 Dimethicone zeichnet sich durch die folgende Strukturformel aus 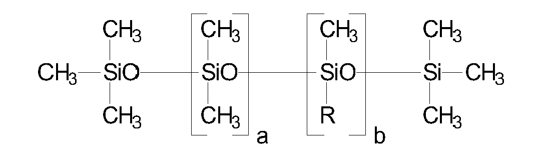 worin R = -CH₂CH₂CH₂O(EO)ₘ(PO)ₙR' und R' = -CH₃ oder -H darstellen.

PEG/PPG-19/19 Dimethicone kann vorteilhaft im Sinne der vorliegenden Erfindung auch in Form kommerziell erhältlicher öliger Vormischungen zur Anwendung kommen, beispielsweise in Mischungen mit Cyclomethicone. Eine solche vorteilhafte Vormischung ist beispielsweise unter der Handelsbezeichnung Dow Corning BY 11-030 bei der Fa. Dow Corning erhältlich.

Die erfindungsgemäßen Pigmente können anorganisch oder organisch sein.

Bevorzugt im Sinne der vorliegenden Erfindung sind Pigmentmischungen aus Weiß-Pigmenten (z. B. Kaolin, Titandioxid oder Zinkoxid) und anorganischen Farbpigmenten (z. B. EisenoxidPigmente, Chromoxide), wobei die Pigmente beschichtet ("gecoatet") oder unbeschichtet vorliegen können. Unter den Farbpigmenten sind Eisenoxide besonders bevorzugt.

Weißpigmente sind Pigmente, deren optische Wirkung vorwiegend auf nicht-selektiver Lichtstreuung beruht (siehe auch DIN 55944: 2003-11). Von den chemisch oft sehr ähnlichen Füllstoffen unterscheiden sich anorganische Weißpigmente vor allem durch ihre im Allgemeinen höhere Brechzahl und - damit verbunden - ihr höheres Streuvermögen sowie nach DIN 55943: 2001-10 durch ihre Anwendung.

Erfindungsgemäß bevorzugte Weißpigmente zeigen keine Absorption im Bereich des sichtbaren Lichts, dafür aber ein hohes Streuvermögen, welches ein hohes Deckvermögen zur Folge hat. Das Streuvermögen ist umso größer, je größer die Differenz zwischen der Brechzahl des Weißpigmentes und der des umgebenden Mediums ist.

Erfindungsgemäß vorteilhafte Weißpigmente sind Titandioxide (Brechzahlen: 2,55 für Anatas und 2,75 für Rutil) und Zinkoxide (Brechzahl zwischen 1,95 und 2,1). Besonders bevorzugt ist Titandioxid.

Vorteilhaft im Sinne der vorliegenden Erfindung können das oder die Pigmente auch aus der Gruppe der Effektpigmente gewählt werden, welche der kosmetischen Zubereitung neben der reinen Farbe eine zusätzliche Eigenschaft - wie z. B. eine Winkelabhängigkeit der Farbe (changieren, Flop), Glanz (nicht Oberflächenglanz) oder Textur - verleihen. Solche Effektpigmente werden erfindungsgemäß vorteilhaft zusätzlich zu einem oder mehreren Weiß- und/oder Farbpigmenten eingesetzt.

Die bedeutendste Gruppe der Effektpigmente stellen die Glanzpigmente dar, zu denen nach DIN 55944: 2003-11 die Metalleffektpigmente und die Perlglanzpigmente gehören. Einige spezielle Effektpigmente lassen sich diesen beiden Gruppen nicht zuordnen, z. B. plättchenförmiges Graphit, plättchenförmiges Eisenoxid und mikronisiertes Titandioxid, wobei mikronisiertes Titandioxid keinen Glanzeffekt, sondern einen winkelabhängigen Lichtstreueffekt erzeugt. Bei den Glanzpigmenten nach DIN 55943: 2001-10 handelt es sich vorwiegend um plättchenförmige Effektpigmente. Parallel orientiert zeigen Glanzpigmente einen charakteristischen Glanz. Die optische Wirkung von Glanzpigmenten beruht auf der gerichteten Reflexion an metallischen Teilchen (Metalleffektpigmente), an transparenten Teilchen mit hoher Brechzahl (Perlglanzpigmente) oder auf dem Phänomen der Interferenz (Interferenzpigmente) (DIN 55944: 2003-11).

Beispiele für erfindungsgemäß bevorzugte handelübliche Effektpigmente sind: Timiron® von Merck , Iriodin® von Merck (Perl- und Farbglanzpigmente für dekorative technische Anwendungen), Xirallic® von Merck (farbintensive Kristalleffektpigmente).

Ferner können die erfindungsgemäßen Zubereitungen vorteilhaft auch organische Farbpigmente enthalten, d. h. organische Farbstoffe, welche in der Zubereitung praktisch unlöslich sind. Nach DIN 55944: 1990-04 können organische Pigmente nach chemischen Gesichtspunkten in Azopigmente und polycyclische Pigmente sowie nach farblichen Gesichtspunkten in Bunt- oder Schwarzpigmente eingeteilt werden. Organische Weißpigmente sind ohne praktische Bedeutung.

Die Pigmente können vorteilhaft im Sinne der vorliegenden Erfindung auch in Form kommerziell erhältlicher öliger oder wässriger Vordispersionen zur Anwendung kommen.

Es ist ferner vorteilhaft im Sinne der vorliegenden Erfindung, wenn die erfindungsgemäße Zubereitung einen oder mehrere Farbstoffe enthält.

Die Farbstoffe können sowohl synthetischen als auch natürlichen Ursprungs sein.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9) sowie Lauroyl Lysine, Polymethylsilesquioxane, Polymethylmethacrylate, Polymethylmethacrylate Crosspolymer, Nylon, Talkum, beschichtetes Talkum, z.B. mit Dimethicone und Trimethylsiloxysilicate, Mica, Silica

Feine Fältchen sieht man, weil sich die Lichtreflexion auf der Hautoberfläche von der in den Faltensenken unterscheidet. Es ist daher vorteilhaft im Sinne der vorliegenden Erfindung, lichtstreuende Pigmente einzusetzen, die die Faltentiefe optisch reduzieren, indem sie den Anteil an diffus reflektiertem Licht in der Falte reduzieren, wodurch die Haut glatter und natürlicher aussieht. Auf diese Weise lässt sich auch übermäßiger Glanz der Haut optisch reduzieren. Vorteilhaft zu diesem Zweck zu verwenden ist speziell beschichtetes Silica - z. B. ganz besonders vorteilhaft Silica LDP (Silica + Titanium Dioxide + Iron Oxides), welches unter dem Handelsnamen Ronasphere LDP von Merck erhältlich ist.

Die erfindungsgemäßen Zubereitungen können vorteilhaft ferner einen oder mehrere weitere (Silicon-) Emulgatoren enthalten, bevorzugt Emulgatoren mit einem HLB-Wert ≤ 8, insbesondere wenn sie in Form von W/S-Emulsionen vorliegen.

Erfindungsgemäß können der oder die weiteren Siliconemulgatoren vorteilhaft aus der Gruppe der grenzflächenaktiven Substanzen aus der Gruppe der Alkylmethiconcopolyole und/oder Alkyl-Dimethiconcopolyole gewählt werden, insbesondere aus der Gruppe der Verbindungen, welche gekennzeichnet sind durch die folgende chemische Struktur: 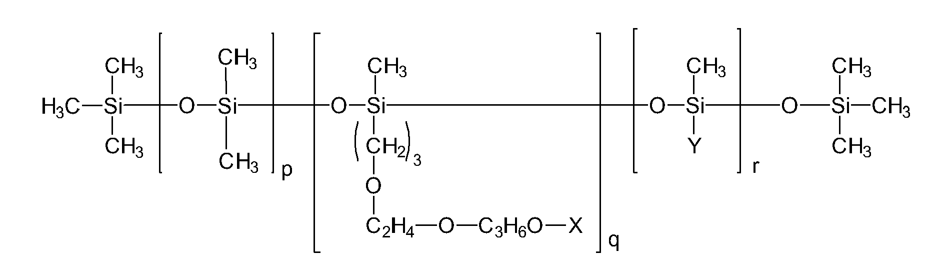 bei welcher X und Y unabhängig voneinander gewählt werden aus der Gruppe H sowie der verzweigten und unverzweigten Alkylgruppen, Acylgruppen und Alkoxygruppen mit 1 - 24 Kohlenstoffatomen, p eine Zahl von 0 - 200 darstellt, q eine Zahl von 1 - 40 darstellt, und r eine Zahl von 1 - 100 darstellt.

Ein Beispiel für besonders vorteilhaft im Sinne der vorliegenden Erfindung zu verwendende Siliconemulgatoren sind Dimethiconcopolyole, welche von der Gesellschaft Degussa AG unter den Warenbezeichnungen ABIL® B 8842, ABIL® B 8843, ABIL® B 8847, ABIL® B 8851, ABIL® B 8852, ABIL® B 8863, ABIL® B 8873 und ABIL® B 88183 verkauft werden.

Ein weiteres Beispiel für besonders vorteilhaft im Sinne der vorliegenden Erfindung zu verwendende Siliconemulgatoren ist das Cetyl PEG/PPG-10/1 Dimethicone, welches von der Gesellschaft Degussa AG unter der Warenbezeichnung ABIL® EM 90 verkauft wird.

Ein weiterer besonders vorteilhaft im Sinne der vorliegenden Erfindung zu verwendender Siliconemulgator ist das Cyclomethicon Dimethiconcopolyol, welches von der Gesellschaft Goldschmidt AG unter der Warenbezeichnung ABIL® EM 97 verkauft wird.

Weiterhin hat sich als ganz besonders vorteilhaft der Emulgator Laurylmethiconcopolyol herausgestellt, welcher unter der Warenbezeichnung Dow Corning® 5200 Formulation Aid von der Gesellschaft Dow Corning Ltd. erhältlich ist.

Die Gesamtmenge an (Silicon-) Emulgatoren in der erfindungsgemäßen kosmetischen Zubereitung wird vorteilhaft aus dem Bereich von 1 bis 10 Gew.-%, besonders bevorzugt von 2 bis 6 Gew.-% gewählt, jeweils bezogen auf das Gesamtgewicht der Zubereitungen, wobei mindestens einer der Emulgatoren das PEG/PPG-19/19 Dimethicone ist.

Die kosmetischen Zubereitungen gemäß der Erfindung können ferner vorteilhaft einen oder mehrere grenzflächenaktive Polyether enthalten, insbesondere wenn die Zubereitungen in Form von W/S-Emulsionen vorliegen:

Vorteilhaft im Sinne der vorliegenden Erfindung sind die in den Chemical Abstracts mit der Registraturnummer 78336-31-9 bezeichneten Polyether, welche die chemische Bezeichnung Polyethylenglycoldi(polydodecylenglycol)ether tragen und beispielsweise als PEG-45 (dodecyl glycol) copolymer unter der Marke Elfacos® ST 9 (mittleres Molekulargewicht ca. 4 000 g/mol) bzw. als PEG-22 (dodecyl glycol) copolymer unter der Marke Elfacos® ST 37 (mittleres Molekulargewicht ca. 2 300 g/mol) von der Gesellschaft Akzo Nobel Chemicals GmbH verkauft werden.

Die Gesamtmenge an den grenzflächenaktiven Polyethern in den fertigen kosmetischen Zubereitungen wird vorteilhaft aus dem Bereich von 0,1 bis 30 Gew.-%, bevorzugt 0,25 bis 5,0 Gew.-% insbesondere 0,75 bis 3,5 Gew.-% gewählt, jeweils bezogen auf das Gesamtgewicht der Zubereitungen.

Es ist ferner vorteilhaft im Sinne der vorliegenden Erfindung, Polyethylenglycol-30-Dipolyhydroxystearat (PEG-30-Dipolyhydroxystearat), welches von der Gesellschaft Uniqema unter der Handelsbezeichnung ARLACEL® P135 verkauft wird, als grenzflächenaktive Substanz zu verwenden, insbesondere wenn die Zubereitung in Form einer Wasser-in-Öl-Emulsion vorliegt. Darüber hinaus kann PEG-30-Dipolyhydroxystearat - ebenso wie andere W/O-Emulgatoren - aber auch vorteilhaft als Filmbildner eingesetzt werden, z. B. um die Abriebfestigkeit (Transfer-resistenz) der Zubereitungen zu verbessern bzw. um Long-lasting Effekte zu erreichen.

Die erfindungsgemäße Zubereitung kann vorteilhaft auch in Form einer Wasser-in-Öl-Emulsion (W/O-Emulsion) vorliegen. In diesem Fall sind die folgenden Emulgatoren erfindungsgemäß bevorzugt:

| **Handelsname** | **INCI-Name** |
|---|---|
| Lameform TGI | Polyglyceryl-3 Diisostearate |
| Isolan Gl 34 | Polyglyceryl-4 Isostearate |
| Dehymuls PGPH | Polyglyceryl-2 Dipolyhydroxystearate |
| Arlacel P 135 | PEG-30 Dipolyhydroxystearate |
| Eucerit PA | Lanolin Alcohol |
| Atlas G-1 049 | PEG-40 Sorbitan Perisostearate |
| Abil EM 90 | Cetyl Dimethicone Copolyol |
| Arlacel 989 | PEG-7 Hydrogenated Castor Oil |
| Elfacos ST 9 | PEG 45/Dodeceyl Glycol Copolymer |
| Elfacos ST 37 | PEG 22/Dodeceyl Glycol Copolymer |
| Isostearinsäure PP | Pentaerythrityl Isostearate |
| Imwitor 780 K | Isostearyl Diglyceryl Succinate |
| Arlacel 987 | Sorbitan Isostearate |
| Hostacerin DGI | Polyglyceryl-2 Sesquiisostearate |
| Tegin ISO | Glyceryl Isostearate |
| Arlacel 60 | Sorbitan Stearate |
| Tegin M | Glyceryl Stearate |
| Arlantone G | PEG-25 Hydrogenated Castor Oil |
| Arlantone T | PEG-40 Sorbitan Peroleate |
| Arlacel 80 | Sorbitan Oleate |
| | Cera Microcristallina + Paraffinum Liquidum + Ozokerite+ Hydrogenated Castor Oil + Glyceryl Isostearate + Polyglyceryl-3 Oleate |
| Atlas G-1049 | PEG-40 Sorbitan Perisostearate |
| ABIL WS 08 | Cetyl Dimethicone Copolyol + Hexyl Laurate + Polyglyceryl-3 Oleate + Cetyl Dimethicone |
| Hostacerin DGO | Polyglyceryl-2 Sesquioleate |
| Abil WE 09 | Cetyl Dimethicone Copolyol (+) Polyglyceryl-4 Isostearate (+) Hexyl Laurate |
| Abil EM 90 | Cetyl PEG/ PPG- 10/1- Dimethicone |
| Abil EM 97 | Dimethicone Copolyol (+) Cyclomethicone |
| Isolan GO 33 | Polyglyceryl-3 Oleate |
| Montanov WO 18 | Isostearyl Alcohol (+) Isostearyl Glucoside |
| Cremophor GO 32 | Polyglyceryl-3 Dioleate |
| Olivem 900 | Sorbitan Oleate |
| Sisterna SP01-C | Sucrose Distearate |
| Sisterna SP10-C | Sucrose Distearate |
| Dehymuls B | Polyglyceryl-3 Diisostearate (+) Glyceryl Oleate |
| Emulsogen SRH | Rapeseed Sorbitols |
| Emulsogen SRO | Rapeseed Sorbitols |
| Rylo PG 11 | Polyglyceryl Dimer Soyate |
| Grindstedt PS 401 (Dermofeel PR) | Polyglyceryl Polyricinoleate |
| Isolan PDI | Polyglyceryl-3 Dimerate |
| Arlacel 986 | Glyceryl Sorbitan Stearate |
| Decaglyn 5-HS | Polyglyceryl-10 Hydroxystearate |

Ganz besonders bevorzugt ist es, wenn der oder die W/O-Emulgatoren gewählt werden aus der Gruppe PEG-30 Dipolyhydroxystearat, Polyglyceryl-3-Diisostearat, PEG-40 Sorbitan Perisostearate.

In einer besonders bevorzugten Ausführungsform weist die Ölphase einen Gehalt an cyclischen und/oder linearen Siliconölen auf bzw. werden cyclische und/oder lineare Siliconöle als alleinige Ölkomponenten verwendet.

Vorteilhaft werden Cyclomethicone, insbesondere Octamethylcyclotetrasiloxan, Cyclomethicone D5 und/oder Cyclomethicone D6 als erfindungsgemäß zu verwendendes Siliconöl eingesetzt. Ein weiteres erfindungsgemäß vorteilhaftes Siliconöl ist Dimethicon (auch: Dimethylpolysiloxan bzw. Polydimethylsiloxan mit der INCl-Bezeichnung Dimethicone).

Aber auch andere Siliconöle sind vorteilhaft im Sinne der vorliegenden Erfindung zu verwenden, beispielsweise Hexamethylcyclotrisiloxan, Poly(methylphenylsiloxan), Phenyltrimethicon (INCI-Bezeichnung: Phenyl Trimethicone, CAS 2116-84-9), Phenyldimethicon, Hexamethylcyclotrisiloxan, Polydimethylsiloxan, Poly(methylphenylsiloxan), Cetyldimethicon (INCl-Bezeichnung: Cyclomethicone, CAS 556-67-2 u. 69430-24-6), Behenoxydimethicon.

Es ist aber auch vorteilhaft, Siliconöle ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Poly-siloxan-polyalkyl-polyether-copolymere wie das Cetyl-Dimethicon-Copolyol.

Erfindungsgemäß besonders vorteilhaft sind Siliconöle sowie Siliconölabmischungen bestehend aus flüchtigen Siliconölen (wie Cyclomethicone) und nicht-flüchtigen Siliconölen (Dimethicone, Phenytrimethicone), insbesondere wenn die Zubereitung in Form einer W/S-Emulsion vorliegt.

Ferner kann die Ölphase einen Gehalt an Dialkylcarbonaten aufweisen, vorteilhaft ist z. B. Dicaprylylcarbonat, beispielsweise das unter der Handelsbezeichnung Cetiol CC bei der Fa. Cognis erhältliche. Ferner kann die Ölphase Triglyceride wie Caprylic / capric Triglyceride, Dialkylether wie Dicaprylylether, natürliche Öle wie Avocado-, Sesam-, Mandel-, Soja-, Aprikosenöl und Kohlenwasserstoffe, linear, verzweigt enthalten

Die Ölphase der erfindungsgemäßen Zubereitungen kann ferner vorteilhaft auch Wachskomponenten enthalten, insbesondere Wachse, deren Schmelzpunkt zwischen 30 und 45 °C, besonders bevorzugt zwischen 30 und 40 °C, liegt.

Besonders vorteilhafte Wachse im Sinne der vorliegenden Erfindung sind die mit den INCI-Bezeichnungen Shea Butter (Butyrospermum Parkii), Myristyl Palmitate, Cetyl Palmitate, Myristyl Lactate, Paraffin Wax, Ceresin, Ozokerite, Ceresin, Microcrystalline Wax, Microcrystalline Wax, Cera Microcrystallina, Hydrogenated Castor Oil, Hydrogenated Cocoglycerides, Glyceryl Laurate, Caprylic/Capric/Isostearic/ Adipic Triglyceride, Glyceryl Caprylate/Caprate/ Isostearate/Adipate (+) Stearinalkonium Hectorite, Octacosanyl Stearate, Hydroxyoctanosyl Hydroxystearate, Cholesterol, C18-38 Alkyl Hydroxystearoyl Stearate, Synthetic Beeswax, Cera Alba, Cera Carnauba, Hydrogenated Soybean Oil, Stearoxy Methicone, Behenoxy Dimethicone, Cetyl Dimethicone, C24-28 Alkyl Methicone, Stearyl Dimethicone, Stearoxy Trimethylsilane + Stearyl Alcohol, Hydrogenated Lecithin.

Besonders bevorzugt sind Shea Butter (Butyrospermum Parkii), Myristylpalmitat, Cetylpalmitat, Myristyllactat.

Es ist vorteilhaft im Sinne der vorliegenden Erfindung, wenn die Zubereitungen einen oder mehrere Glycerinester, insbesondere Glycerinester von α-Hydroxycarbonsäuren und gesättigten Fettsäuren enthalten, wobei die Gesamtmenge der Glycerinester in den fertigen kosmetischen Zubereitungen vorteilhaft aus dem Bereich von 0,1 bis 10,0 Gew.-%, bevorzugt 0,5 bis 6,0 Gew.-% gewählt wird, jeweils bezogen auf das Gesamtgewicht der Zubereitungen.

Die kosmetischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Verdickungsmittel, anfeuchtende und/oder feuchthaltende Substanzen, Füllstoffe, die das Hautgefühl verbessern, oder andere übliche Bestandteile einer kosmetischen Formulierung wie Alkohole, Polyole, Polymere, Schaumstabilisatoren, Elektrolyte, organische Lösungsmittel oder Siliconderivate.

Geeignete Konservierungsmittel im Sinne der vorliegenden Erfindung sind beispielsweise Formaldehydabspalter (wie z. B. DMDM Hydantoin, welches beispielsweise unter der Handelsbezeichnung Glydant^{™} von der Fa. Lonza erhältlich ist), Iodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfasst das Konservierungssystem erfindungsgemäß ferner vorteilhaft auch Konservierungshelfer, wie beispielsweise Octoxyglycerin, Glycine Soja etc.

Obwohl die genannten Konservierungsmittel an sich in den erfindungsgemäßen Zubereitungen vorteilhaft eingesetzt werden können, sind ganz besonders bevorzugt solche Zubereitungen, welche frei von Parabenen und/oder frei von Formaldehydabspaltern sind.

Vorteilhafte Komplexbildner im Sinne der vorliegenden Erfindung sind beispielsweise EDTA, [S,S]-Ethylendiamindisuccinat (EDDS), welches beispielsweise unter der Handelsbezeichnung Octaquest von der Fa. Octel erhältlich ist, Pentanatrium-Ethylendiamintetramethylenphosphonat, welches z. B. unter dem Handelsnamen Dequest 2046 von der Fa. Monsanto erhältlich ist und/oder Iminodibersteinsäure, welche u. a. von der Fa. Bayer AG unter den Handelsnamen Iminodisuccinat VP OC 370 (ca. 30% ige Lösung) und Baypure CX 100 fest erhältlich ist.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure (Vitamin C) und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E oder Vitamin C und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. Ginkgo Biloba bzw. Ginkgo-Extrakte, Hyaluronsäure, Collagen, alpha-Liponsäure, Phytoen, D-Biotin, Coenzym Q10, alpha-Glucosylrutin, Carnitin, Carnosin, natürliche und/oder synthetische Isoflavonoide, Kreatin, Taurin und/oder β-Alanin sowie 8-Hexadecen-1,16-dicarbonsäure (Dioic acid, CAS-Nummer 20701-68-2; vorläufige INCI-Bezeichnung Octadecendioic acid).

Erfindungsgemäße Zubereitungen, welche z. B. bekannte Faltenfüllwirkstoffe wie Hyaluronsäure und/oder Derivate und dergleichen enthalten, eignen sich insbesondere vorteilhaft zur kosmetischen Behandlung von Hautveränderungen, wie sie z. B. bei der Hautalterung auftreten (wie Ausbildung von Trockenheitsfältchen, Schlaffheit und Ausbildung von Falten und Fältchen). Weiterhin vorteilhaft eignen sich Collagen bzw. Wirkstoffe, die die Collagensynthese steigern, wie Ginkgo Biloba bzw. Ginkgo-Extrakte, Vitamin C, Creatin, um die Haut zu festigen und zu straffen.

Vorteilhaft ist ferner die Verwendung von erfindungsgemäßen Zubereitungen, welche Ascorbinsäure und/oder Derivate und/oder UV-Filtersubstanzen enthalten, zur kosmetischen Behandlung von Hautalterungserscheinungen (Ausbildung von Trockenheitsfältchen, Schlaffheit und Ausbildung von Falten und Fältchen).

Die Wasserphase der Zubereitungen gemäß der vorliegenden Erfindung kann vorteilhaft übliche kosmetische Hilfsstoffe enthalten, wie beispielsweise Alkohole, insbesondere solche niedriger C-Zahl, vorzugsweise Ethanol und/oder Isopropanol, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Magnesium Aluminium Silikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole [von der Fa. Noveon], beispielsweise Carbopole der Typen 980, 981, 1382, 2984, 5984, ETD 2020, ETD 2050, Pemulen TR-1 oder -2, Ultrez 10, jeweils einzeln oder in Kombination.

Auch sog. Moisturizer sind vorteilhaft zu verwenden. Als Moisturizer werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.
Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff.

Es ist erfindungsgemäß bevorzugt, den Gehalt an UV-Filtersubstanzen (eine oder mehrere Verbindungen) kleiner als 5 Gew.-%, insbesondere kleiner als 2 Gew.-% zu wählen, jeweils bezogen auf das Gesamtgewicht der Zubereitung.

Für den Fall, dass ein Gehalt an UV-Filtersubstanzen gewünscht ist, werden diese vorteilhaft aus einer oder mehreren der folgenden Gruppen gewählt: UV-A-, UV-B- und/oder Breitbandfiltersubstanzen sowie organische und/oder anorganische Pigmente als UV-Filtersubstanzen. Anorganische Pigmente wie Titandioxid besonders bevorzugt Titandioxid AQ-IP der Fa. Unichema.

Besonders vorteilhafte UV-Filtersubstanzen im Sinne der vorliegenden Erfindung sind:
- Dibenzoylmethanderivate, insbesondere das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex® 9020 verkauft wird;
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonat (CAS-Nr.: 180898-37-7), welches beispielsweise unter der Handelsbezeichnung Neo Heliopan AP bei Symrise erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Symrise erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird;
- Benzol-1,4-di(2-oxo-3-bornylidenmethyl-10-sulfonsäure) hat die INCl-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)-benzolsulfonsäure, 2-Methyl-5-(2-oxo-3-bornylidenmethyl)sulfonsäure und deren Salze.
- Benzoxazol-Derivate, wie z. B. das 2,4-bis-[5-1 (dimethylpropyl)benzoxazol-2-yl-(4-phenyl)-imino]-6-(2-ethylhexyl)-imino-1,3,5-triazin mit der CAS Nr. 288254-16-0, welches bei 3V Sigma unter der Handelsbezeichnung Uvasorb® K2A erhältlich ist;
- Hydroxybenzophenone, z. B. der 2-(4'-Diethylamino-2'-hydoxybenzoyl)-benzoesäurehexylester (auch: Aminobenzophenon), welcher unter der Handelsbezeichnung Uvinul A Plus bei der Fa. BASF erhältlich ist;
- Triazinderivate, wie z. B. 2,4-Bis-{[4-(2-Ethyl-hexyloxy)-2-hydroxy]-phenyl}-6-(4-methoxyphenyl)-1,3,5-triazin (INCI: Bis-Ethylhexyloxylphenol Methoxyphenyl Triazin), welches unter der Handelsbezeichnung Tinosorb® S bei der CIBA-Chemikalien GmbH erhältlich ist;
- Dioctylbutylamidotriazon (INCI: Diethylhexyl Butamido Triazone), welches unter der Handelsbezeichnung UVASORB HEB bei Sigma 3V erhältlich ist;
- 4,4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCl: Ethylhexyl Triazone), welches von der BASF Aktiengesellschaft unter der Warenbezeichnung UVINUL® T 150 vertrieben wird;
- 2-[4,6-Bis(2,4-dimethylphenyl)-1,3,5-triazin-2-yl]-5-(octyloxy)phenol (CAS Nr.: 2725-22-6).
- Benzotriazole, wie z. B. 2,2'-Methylen-bis-(6-(2H-benzotriazol-2-yl)-4-(1,1,3,3-tetramethylbutyl)-phenol) (INCI: Methylene Bis-Benztriazolyl Tetramethylbutylphenol), welches z. B. unter der Handelsbezeichnung Tinosorb® M bei der CIBA-Chemikalien GmbH erhältlich ist;
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Benzalmalonsäure, vorzugsweise 4-Methoxybenzalmalonsäuredi(2-ethylhexyl)-ester;
- Ester der Zimtsäure, vorzugsweise 4-Methoxyzimtsäure(2-ethylhexyl)ester (Octylmethoxycinnamate), 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- an Polymere gebundene UV-Filter sowie
- Ethylhexyl-2-cyano-3,3-diphenylacrylat (Octocrylen), welches von BASF unter der Bezeichnung Uvinul^{®} N 539 T erhältlich ist.

Es ist ferner vorteilhaft im Sinne der vorliegenden Erfindung, wenn die Zubereitung einen oder mehrere Filmbildner bzw. Polymere enthält, insbesondere um einen nachvollziehbaren Straffungseffekt auf der Haut zu erzielen, der durch deren Filmbildungseigenschaften hervorgerufen werden kann. Gleichzeitig dienen die Filmbildner zur Fixierung der Pigmente auf der Haut, insbesondere um einen langanhaltenden Effekt und eine Transfer-Resistenz zu erzielen.

Vorteilhafte Filmbildner im Sinne der vorliegenden Erfindung sind z. B. Trimethylsiloxysiliacte (beispielsweise auch in Abmischung mit Dimethicone), Polyurethane (z. B. die Avalure® -Typen von Goodrich), Dimethicone Copolyol Polyacrylate (Silsoft Surface® von der Witco Organo Silicones Group), PVP/VA (VA = Vinylacetat) Copolymer (Luviscol VA 64 Powder der BASF), C₂₀₋₄₀ Carbonsäure mit Polyethylen (Performacid 350 von der Fa. New Phase Technologies) sowie Filmbildner aus der Gruppe der Polymere auf Basis von Polyvinylpyrrolidon (PVP):

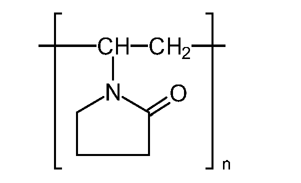

Besonders bevorzugt werden Copolymere des Polyvinylpyrrolidons eingesetzt, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind, sowie das Tricontayl PVP und dergleichen mehr.

Insbesondere bevorzugt sind wasserlösliche Polymere wie Vinylpyrrolidon/Vinylacetat- (VP/VA-) Copolymere und Natriumpolystyrensulfonat.

Es ist vorteilhaft im Sinne der vorliegenden Erfindung die Konzentrationen an Filmbildnern (eine oder mehrere Verbindungen) aus dem Bereich von 0,1 bis 5 Gew.-%, besonders bevorzugt wie 0,1 bis 3 Gew.-% - jeweils bezogen auf das Gesamtgewicht der Zubereitungen - zu wählen.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele:

### W/O- bzw. W/S Foundations

| | **1** | **2** | **3** | **4** | **5** | **6** |
|---|---|---|---|---|---|---|
| | **Gew.-%** | **Gew.-%** | **Gew.-%** | **Gew.-%** | **Gew.-%** | **Gew.-%** |
| Wasser | ad 100 | ad 100 | ad 100 | ad 100 | ad 100 | ad 100 |
| Zitronensäure | | 0.1 | | | | |
| Alcohol Denat. | | | 10 | | 2,5 | 5 |
| Cetyl PEG/PPG-10/1 Dimethicon | | 4 | 1,5 | 2 | | |
| **PEG/PPG-19/19 Dimethicone** | 2 | 0,5 | 1,5 | 1 | 1,5 | 3 |
| PEG-30 Dipolyhydroxystearate | | | 0,5 | | | |
| Magnesium Sulfat | | | 1 | | | |
| Disteardimonium Hectorit | 0,5 | 0,5 | 0,25 | 0,25 | 0,25 | 0,5 |
| Natrium Chlorid | 2 | 2 | | 2 | 2 | 2 |
| Propylen Carbonat | 0,12 | 0,12 | 0,05 | 0,06 | 0,06 | 0,12 |
| Caprylic/Capric Triglycerid | | | | | | 4 |
| Dicaprylyl Ether | | | | | | 4 |
| Dicaprylyl Carbonate | 3 | | | 5 | 8 | |
| Dimethicon | | 3 | | | | 2 |
| Cyclomethicon | 15 | 17 | 30 | 19 | 13 | 15 |
| Dimethicon + Trimethylsiloxysilicat | | | | 3,5 | 3,5 | |
| Dimethicon + Dimethicon Crosspolymer | 6 | 4 | 6 | | | |
| Cyclomethicon + Dimethiconol | | 2 | | | | |
| Avocadoöl | | | 2 | | | |
| Aprikosenkernöl | | | 1 | | | |
| Squalan | | 4 | | 2 | 0,5 | |
| Lecithin | | | 1 | | | |
| Shea Butter | 1 | | | | | 1 |
| Myristyl Lactat | 1 | | | | | |
| Silica | | | 1 | | | |
| Aluminum Starch Octenylsuccinat | 3 | | 2 | | | 1 |
| Lauroyl Lysin | 1 | 3 | 1 | 2 | 2 | 4 |
| Talc | | 1 | | | | |
| Methyl Methacrylat Crosspolymer | | 1 | | | | |
| Nylon | | 2 | | 4 | 8 | |
| Mit Dimethylimidazolidinon modifizierte Reisstärke | | | 2 | | | |
| [Talkum beschichtet mit Dimethicon + Trimethylsiloxysilicat] | | | | 1 | | |
| Polymethylsilsesquioxan | | | 3 | | | 2 |
| VP/VA Copolymer | | 0,1 | | | | 0,2 |
| VP/Hexadecen Copolymer | | | | 0,5 | | 1,5 |
| Sodium Polystyren Sulfonat | 0,5 | 1 | 1 | | 0,4 | 0,5 |
| Titandioxid (Cl 77891) | 9 | 6 | 7 | 6 | 7 | 5 |
| Farbpigmente (Cl77492, 77491,77499,77007) | 4,5 | 5 | 6 | 7,5 | 5,5 | 6 |
| Beschichtetes Silica 2 | 3 | 2 | | 1 | 0,5 | |
| Effektpigmente (z.B. beschichtetes Mica) 3 | | 5 | 0,5 | | | 3,5 |
| Glycerin | 10 | 5 | 7 | 12,5 | 5 | 5 |
| Natrium Hyaluronat | | 1 | | | | 2 |
| Ethylhexyl Methoxycinnamat + BHT | 1 | 5 | | | | 1 |
| Titanium Dioxid | 3 | | 1 | | | 1 |
| Wasser + Glycerin + Lecithin + Butylene Glycol + Sodium Styrene/Acrylates Copolymer | 3 | 5 | | | | 0,5 |
| Ginkgo Biloba | | | 2 | | | |
| Vitamin E Acetat | 1,5 | 1 | 1,5 | 0,5 | 0,5 | 1,5 |
| Vitamin A Palmitat | 0,1 | | 0,5 | | | |
| Ubiquinon, Q10 | | 0,025 | | | 0,2 | |
| Biosaccharide Gum-1 | | | | | | 1 |
| Vitamin C Phosphate | | 0,25 | | 0,1 | | 0,1 |
| Calcium Pantothenat | | 0,5 | | | | |
| Glycin | 0,1 | | | | 0,7 | |
| Phenoxyethanol + Paraben | 1 | | 0,5 | | | 0,7 |
| Tetrasodium Iminodisuccinat | 0,2 | | | | | |
| Diazolidinyl Urea | 0,2 | 0,3 | | 0,3 | 0,3 | |

| | | | | | | |
|---|---|---|---|---|---|---|
| ^{2z.B.} Ronasphere LDP von Merck (Silica + Titanium Dioxide + Iron Oxides) ^{3z.B..} Timiron von Merck (Mica & Cl 77891) | | | | | | |

## Patentansprüche

1. Kosmetische Zubereitung vom Typ Wasser-in-Öl, welche
a) 0,5 bis 3 Gew.-% PEG/PPG-19/19 Dimethicone,
b) 5 bis 15 Gew.-% eines oder mehrerer Pigmente sowie enthält.

2. Zubereitung nach Anspruch 1, **dadurch gekennzeichnet, dass** sie weitere Emulgatoren mit einem HLB-Wert ≤ 8 enthält, wobei die Gesamtmenge der Emulgatoren - bezogen auf das Gesamtgewicht der Zubereitungen - aus dem Bereich von 2 bis 6 Gew.-% gewählt wird.

3. Zubereitung nach einem der vorhergehenden Ansprüche, **dadurch gekennzeichnet, dass** als Pigment Pigmentmischungen aus Weiß-Pigmenten und anorganischen Farbpigmenten verwendet werden.

4. Zubereitung nach Anspruch 3, **dadurch gekennzeichnet, dass** als Farbpigment Eisenoxid verwendet wird.

5. Zubereitung nach einem der Ansprüche 3 oder 4, **dadurch gekennzeichnet, dass** als Weiß-Pigment Titandioxid verwendet wird.

6. Zubereitung nach einem der vorhergehenden Ansprüche, **dadurch gekennzeichnet, dass** als Pigmente Effektpigmente verwendet werden.

7. Zubereitung nach einem der vorhergehenden Ansprüche, **dadurch gekennzeichnet, dass** sie einen oder mehrere Füllstoffe enthält.

8. Zubereitung nach einem der vorhergehenden Ansprüche, **dadurch gekennzeichnet, dass** die Ölphase einen Gehalt an cyclischen und/oder linearen Siliconölen aufweist.

9. Zubereitung nach einem der vorhergehenden Ansprüche, **dadurch gekennzeichnet, dass** cyclische und/oder lineare Siliconöle als alleinige Ölkomponenten verwendet werden.

10. Zubereitung nach einem der vorhergehenden Ansprüche, **dadurch gekennzeichnet, dass** sie Wachse enthält, deren Schmelzpunkt zwischen 30 und 45 °C liegt.
